## Europäisches Patentamt

## European Patent Office

## Office européen des brevets

(11) Publication number: **0 100 517**
**B1**

# EUROPEAN PATENT SPECIFICATION

(45) Date of publication of patent specification: **27.12.90**

(51) Int. Cl.⁵: **A 61 B 1/00, A 61 B 1/12**

(21) Application number: **83107346.5**

(22) Date of filing: **26.07.83**

(54) Optical fiber sensor.

(30) Priority: **31.07.82 JP 134055/82**
**23.02.83 JP 29963/83**
**24.02.83 JP 30579/83**
**09.03.83 JP 39683/83**
**01.04.83 JP 58423/83**
**19.04.83 JP 68860/83**
**18.03.83 JP 40540/83 u**

(43) Date of publication of application:
**15.02.84 Bulletin 84/07**

(45) Publication of the grant of the patent:
**27.12.90 Bulletin 90/52**

(84) Designated Contracting States:
**DE FR GB IT NL**

(56) References cited:
**EP-A-0 097 934        GB-A-2 108 391**
**DE-A-1 302 809        US-A-2 843 112**
**FR-A-1 386 962        US-A-3 089 484**

**FIBER OPTICS - Principles and Applications, by**
**N.S. Kapany, Academic Press 1967, pages**
**177-180**

(73) Proprietor: **SUMITOMO ELECTRIC INDUSTRIES**
**LIMITED**
**No. 15, Kitahama 5-chome Higashi-ku**
**Osaka-shi Osaka 541 (JP)**

(72) Inventor: **Kimizo, Ono Osaka Works of**
**Sumitomo Elec**
**Industries, Ltd 1-3 Shimaya 1-chome**
**Konohana-ku Osaka-shi Osaka (JP)**
Inventor: **Koichi, Tsuno Osaka Works of**
**Sumitomo Elec**
**Industries, Ltd 1-3 Shimaya 1-chome**
**Konohana-ku Osaka-shi Osaka (JP)**
Inventor: **Mitsuru, Nishikawa Osaka Works of**
**Sumitomo Elec**
**Industries, Ltd 1-3 Shimaya 1-chome**
**Konohana-ku Osaka-shi Osaka (JP)**

(74) Representative: **Patentanwälte Grünecker,**
**Kinkeldey, Stockmair & Partner**
**Maximilianstrasse 58**
**D-8000 München 22 (DE)**

(56) References cited:
**OPTICS, by E. Hecht and A. Zajac, Addison-**
**Wesley Publishing Company, 1980, pages 123-**
**125**

Courier Press, Leamington Spa, England.

## Description

The present invention relates to an optical fiber sensor according to the generic clause of claim 1.

An optical fiber sensor of this type can be used as an instrument which is inserted into a cavity of a living body to measure the attraction, absorption and pressure of the liquid in the cavity. This fiber sensor can also be used as an instrument for observing the inside of the cavity when it is constituted by an image fiber for transmitting an image from the cavity, a fiber for transmitting illumination light to the cavity and an outer cover.

With such an optical fiber sensor the use efficiency of the available cross-sectional area for illumination is limited as the outer diameter of such a sensor should not exceed 2.3 mm. This limitation has been a problem in providing an observing device in the form of an optical fiber sensor of high efficiency.

An optical fiber sensor in accordance with the generic clause of claim 1 is disclosed in US—PS 3,089,484.

In order to enhance the efficiency for illumination it is known from "Fiber Optics — Principles and Applications", Academic Press, N. S. Kapany, 1967, pages 177 to 180, to use a fiberscope provided with a light source for illumination purposes, the light of which is directly guided to the entrance of the light guide, the fiberscope being further provided with a concave reflecting mirror to reflect light onto the entrance which otherwise would escape without striking the entrance of the light guide.

However, the illumination efficiency of such a device is still not fully satisfactory.

It is, therefore, to be regarded as an object underlying the present invention to suggest an optical fiber sensor having a high illumination efficiency.

This task is solved with an optical fiber sensor having the features of claim 1.

Preferred embodiments are disclosed in the depending claims.

The invention will now be described with reference to the accompanying drawings wherein:

Fig. 1 is a transverse cross-section of an embodiment of the optical fiber sensor according to the present invention, in which a light guide and an image transmitting fiber bundle are integrally formed;

Fig. 2 is a schematic diagram of an embodiment of the whole of the optical fiber sensor according to the present invention, showing the light coupling system;

Fig. 3 is a front view of the first concave mirror of Fig. 2;

Fig. 4 is an enlarged longitudinal cross-section of the rearward end portion of the light and image conducting part of the optical fiber sensor of Fig. 2;

Fig. 5 is a transverse cross-section taken along the XXIV—XXIV line of Fig. 4.

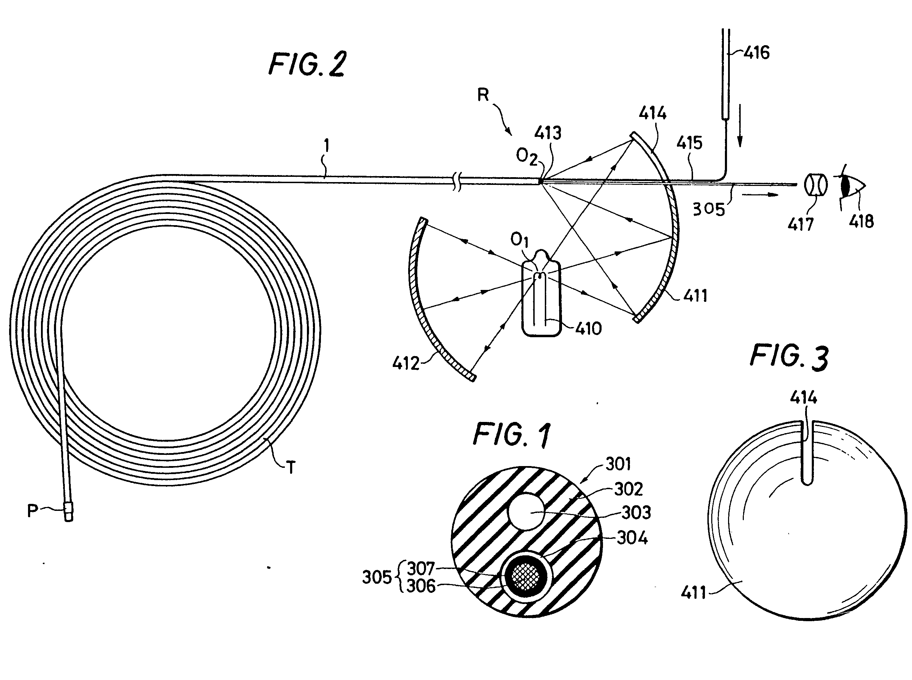

Fig. 1 is a cross-section of an optical fiber sensor 301.

Said optical fiber sensor 301 comprises, as a main part, a transparent light guide 302 having a circular cross-section. The light guide 302 is made of a flexible transparent material such as PMMA (polymethylmethacrylate) resin.

The light guide 302 is produced by molding a plastic material extruded from a suitable die, and at this time a hole 303 for fluid and another hole 304 for passing an image transmitting optical fiber bundle therethrough are simultaneously formed therewithin. The fluid passage 303 is for pouring/transmitting carbonic acid gas or physiological saline solution.

An image transmitting optical fiber bundle 305 is made to pass through the hole 304. The image transmitting fiber bundle 305 is composed of an image transmitting portion 306 including a bundle of a plurality of glass and silica fibers, and of a light absorbing layer 307 covering the outer periphery of the portion 306.

As an example, the outer diameter of the optical fiber sensor 301 is 3 mm, the diameter of the image transmitting fiber bundle 305 is 0.6 mm, and the diameter of the image transmission portion 306 for substantially transmitting picture images is 0.4 mm. The number of fiber strands (picture elements) of the image transmission portion 306 is 3000.

In such a composite optical fiber sensor 301, the light guide 302 and the image transmitting fiber bundle 305 are not separated from each other but are integrally formed. Accordingly it is impossible to separately take out only the light guide fiber bundle so as to directly connect the same to a light source.

Further, it is difficult to lead light from a light source into the light guide because the light guide is very thin.

The composite optical fiber sensor 301 may be called an image transmitting fiber bundle with an outer peripheral light guide, since it has the light guide 302 at the outer periphery thereof or may be called a light guiding ring-like path, since the light guide 302 is disposed at a ring-like portion at the outer periphery in the cross-sectional view and the fluid passage 303 and the image fiber bundle 305 are disposed at or near the center. Accordingly, the term "light guiding ring-like path" means a light guide which is disposed at the outer periphery of the optical fiber device.

The optical fiber sensor according to the present invention is besides provided with a light coupling system for leading illumination light into the thin light guiding ring-like path.

According to such a light coupling system, a first concave mirror is used to lead light from a light source into an end face of the light guiding ring-like path. The concave mirror is shaped and disposed so as to reflect light from the light source and focus the reflected light on the end face of the light guiding ring-like path.

In order to increase the coupling efficiency, it is also effective to provide another concave mirror, separately from the above-mentioned first concave mirror, for causing light emitted from the light source at the side opposite the fiber sensor to return back to the light source.

The first concave mirror is an elliptical concave surface mirror disposed such that its two focuses are made to coincide with the light source and the end face of the light guide ring-like path respectively.

A spherical concave mirror is suitably used as the second concave mirror.

If it is necessary to pass the liquid pipe and the image fiber bundle in a straight path, it is sufficient to provide a slot in the first concave mirror to allow the pipe and image fiber bundle to pass therethrough.

Fig. 2 is a schematic diagram illustrating the whole of an endoscope provided with a light coupling system.

The endoscope is constituted of an image pickup portion P which approaches the object to be observed to receive the image therefrom, a long transmission portion T, and an image receiving portion R. The invention provides coupling between the light source and the light guide at the image receiving portion R. The structure of each of the image pickup portion P and the transmission portion T may be arbitrarily selected and may include apparatus according to other embodiments of the invention.

A light source such as a lamp 410 is placed in the vicinity of the image receiving portion R. The first and second concave mirrors 411 and 412 are placed behind and ahead of the light source 410, respectively.

The end face 413 of the light guiding ring-like path (light guide 302), the light source 410 and the first concave mirror 411 are disposed such that the light emitted from the light source 410 is reflected by the first concave mirror and focused on the end face 413 of the light guiding ring-like path.

The center of a filament of the light source 410 and the end face of the light guiding ring-like path are selected as points $O_1$ and $O_2$, respectively.

It is sufficient to cause a real image of the light source to be produced on the end face 413.

In this example, the filament and light guide were respectively 2 mm × 3 mm in dimension and 3 mm in diameter. One uses as the first concave mirror 411 an ellipsoid of revolution having focuses at the respective points $O_1$ and $O_2$. This is simply referred to hereinafter as an ellipsoidal concave mirror.

An ellipse has a characteristic such that a normal provided at any point on the ellipse always bisects the angle formed by the lines connecting the point and each of the two focuses. Accordingly, an ellipsoidal concave mirror can focus all the light emitted from one focus onto the other focus.

The second concave mirror 412 placed ahead the light source 410 reflects the light from the light source so as to cause it is to reflect back onto the first concave mirror 411. Since this light impinges onto the first concave mirror 411 in the same manner as that emitted from the focal point $O_1$, it is focused on the end surface $O_2$ of the light guiding ring-like light path.

The second concave mirror 412 can thus lead light oppositely emitted from the light source into the light guiding path to thereby increase the light coupling efficiency. Accordingly, a spherical concave mirror with the point $O_1$ as its center is used as the second concave mirror 412.

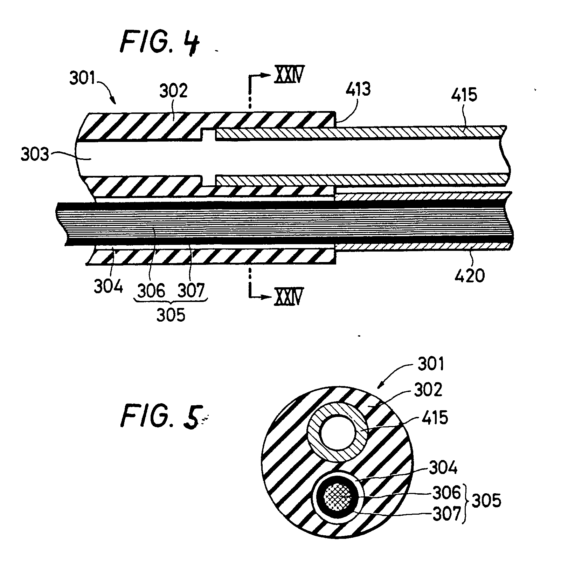

Fig. 4 is an enlarged longitudinal cross-section of the rearward portion of the optical fiber sensor. Fig. 5 is a cross-section along the XXIV—XXIV line in Fig. 4.

A metallic fluid pipe 415 extends from the end face 413 of the light guiding ring-like path 302 and is coupled to a flexible fluid delivery tube 416 (Fig. 2).

The image fiber bundle 305 also extends from the end surface 413 of the light guiding ring-like path. The first concave mirror 411 is formed with a slot 414 so as to allow the image fiber bundle 305 and the fluid pipe 415 to pass therethrough. The image on the end face of the image fiber bundle 305 is observed by the eye 418 through a lens 417 (Fig. 2).

Fig. 3 is a front view of the first concave mirror 411. Since the slot 414 occupies only a small part of the reflecting surface of the mirror, the mirror function is not deteriorated. If the fluid pipe 415 and the image fiber bundle 305 are sufficiently flexible to be bent, the slot 414 may be eliminated.

In Fig. 4, the metal fluid pipe 415 is inserted into the fluid passage 303 of the light guide 302 from the back end face 413. The image fiber bundle 305 is covered with an illumination light reflecting layer 420 on the outer surface of the light absorbing layer 307. This is for shielding the image fiber bundle 305 from light from the strong light source. For example, a metal reflex film such as thin aluminum foil may be used.

According to the present invention, it is possible to cause the illumination light flux from the light source to efficiently impinge on the end face of the light guide 302 disposed at the outer periphery of the image fiber bundle 305 or the like. Even where the light guide 302 has a thin diameter, the illumination flux can be throttled by a concave mirror and the light coupling efficiency is high.

When a slot 414 is formed in the first concave mirror 411, it is easy to replace the image fiber bundle 305 and/or the fluid pipe 415.

Since the second concave mirror 412 is provided at the side opposite the first concave mirror 411 with respect to the light source, the light from the light source is utilized more effectively.

**Claims**

1. An optical fiber sensor, comprising a first light transmission path (302) and a second light transmission path (305), said first light transmission path (302) having therein a longitudinal space (304), and said second light transmission path (305) extending in said space of said first light transmission path in a manner such that light transmission is possible both from one end of said first light transmission path (302) to the other end thereof, and from one end of said second light transmission path (305) to the other end thereof, said optical fiber sensor being provided with optical means (411, 412) for introducing light emitted from a light source (410) into an end-face (413) of said first light transmission path (302), characterized in that said optical means comprise a first (411) and a second (412) concave mirror; said first concave mirror (411) being defined by a part of a rotational elliptical body for introducing illumination light into the first light transmission path (302), wherein the incident end face (413) of the first light transmission path (302) and the light source (410) are respectively located at the focal points ($O_1$, $O_2$) of said rotational elliptical body (411); and said second concave mirror (412) being defined by a spherical surface which is disposed in confronted relation with said first mirror (411) such that its center of curvature is provided at the light source position ($O_1$) so as to reflect light from said light source back to said light source.

2. An optical fiber sensor in accordance with claim 1, wherein said rotational elliptical body (411) comprises a radial slot (414).

3. An optical fiber sensor in accordance with claim 1 or 2, wherein said second light transmission path in the inner portion thereof is constituted by an image transmitting fiber bundle (306).

4. An optical fiber sensor in accordance with claim 3, in which a fluid passageway (303) is provided within the first light transmission path (302).

**Patentansprüche**

1. Ein Optikfasersensor, mit einem ersten Lichttransmissionsweg (302) und einem zweiten Lichttransmissionsweg (305), wobei in dem ersten Lichttransmissionsweg ein Längsraum (304) vorhanden ist, und der zweite Lichttransmissionsweg sich in dem Raum von dem ersten Lichttransmissionsweg in einer solchen Weise erstreckt, daß Lichttransmission sowohl von einem Ende des ersten Lichttransmissionsweges (302) zu dessen anderem Ende als auch von einem Ende des zweiten Lichttransmissionsweges (305) zu dessen anderem Ende möglich ist, und der Optikfasersensor mit optischen Einrichtungen (411, 412) für die Einleitung von von einer Lichtquelle (410) emittiertem Licht in eine Endstirnfläche (413) des ersten Lichttransmissionsweges (302), dadurch gekennzeichnet, daß die optischen Einrichtungen einen ersten (411) und einen zweiten (412) konkaven Spiegel umfassen; wobei der erste konkave Spiegel (411) durch einen Teil von einem rotationselliptischen Körper für die Einführung von Illuminationslicht in den ersten Lichttransmissionsweg (302) gebildet ist, wobei die Einfallsendstirnfläche (413) des ersten Lichttransmissionsweges (302) und die Lichtqelle (410) jeweils in den Brennpunkten ($O_1$, $O_2$) des rotationselliptischen Körpers (411) angeordnet sind; und der zweite konkave Spiegel (412) durch eine sphärische Oberfläche gebildet ist, welche in gegenüberliegender Stellung zu dem ersten Spiegel (411) derart angeordnet ist, daß ihr Krümmungszentrum an der Lichtquellenposition ($O_1$) angeordnet ist, um so Licht aus der Lichtquelle zurück zu der Lichtquelle zu reflektieren.

2. Ein Optikfasersensor nach Anspruch 1, worin der rotationselliptische Körper (411) einen radialen Schlitz (414) umfaßt.

3. Ein Optikfasersensor nach Anspruch 1 oder 2, worin der zweite Lichttransmissionsweg (305) in dessen innerem Teil durch ein bildübertragendes Faserbündel (306) gebildet ist.

4. Ein Optikfasersensor nach Anspruch 3, in welchem ein Flüssigkeitsdurchgangsweg (303) innerhalb des ersten Lichttransmissionsweges (302) vorgesehen ist.

**Revendications**

1. Capteur à fibre optique, comprenant un premier trajet (302) de transmission de lumière et un second trajet (305) de transmission de lumière, le premier trajet (302) ayant un espace longitudinal (304), et le second trajet (305) étant disposé dans l'espace du premier trajet de transmission de lumière de manière que la transmission de lumière soit possible à la fois-d'une première extrémité du premier trajet (302) à son autre extrémité et d'une première extrémité du second trajet (305) à l'autre extrémité de celui-ci, le capteur à fibre optique comportant un dispositif optique (411, 412) destiné à introduire la lumière émise par une source lumineuse (410) par une face d'extrémité (413) du premier trajet (302), caractérisé en ce que le dispositif optique comporte un premier (411) et un second (412) miroir concave, le premier miroir concave (411) étant délimité par une partie d'un corps elliptique de rotation destiné à introduire la lumière d'éclairement dans le premier trajet (302) de transmission de lumière, la face d'extrémité (413) d'incidence du premier trajet (302) et la source lumineuse (410) étant placées respectivement aux foyers ($O_1$, $O_2$) du corps elliptique de rotation (411), et le second miroir concave (412) étant délimité par une surface sphérique disposée en face du premier miroir (411) de manière que son centre de courbure occupe la position de la source lumineuse ($O_1$) afin qu'il renvoie la lumière de la source lumineuse vers cette dernière.

2. Capteur à fibre optique selon la revendication 1, dans lequel le corps elliptique de rotation (411) comporte une fente radiale (414).

3. Capteur à fibre optique selon la revendication 1 ou 2, dans lequel le second trajet de transmission de lumière (305), dans sa partie interne, est constitué par un faisceau (306) de fibres de transmission d'image.

4. Capteur à fibres optique selon la revendication 3, dans lequel un passage (303) de fluide est formé dans le premier trajet (302) de transmission de lumière.

FIG.2

FIG.1

FIG.3

EP 0 100 517 B1

## FIG. 4

## FIG. 5